Europäisches Patentamt

European Patent Office

Office européen des brevets

(19)

(11) Numéro de publication: **0 272 177 B1**

# FASCICULE DE BREVET EUROPEEN

(45) Date de publication du fascicule du brevet:
04.07.90

(51) Int. Cl.⁵: **A61K 31/55**

(21) Numéro de dépôt: 87402614.9

(22) Date de dépôt: 19.11.87

(54) Compositions pharmaceutiques contenant du diltiazem et un inhibiteur de l'enzyme de conversion de l'angiotensine.

(30) Priorité: 20.11.86 FR 8616138

(43) Date de publication de la demande:
22.06.88 Bulletin 88/25

(45) Mention de la délivrance du brevet:
04.07.90 Bulletin 90/27

(84) Etats contractants désignés:
AT BE CH DE FR GB IT LI LU NL SE

(56) Documents cités:
EP-A- 0 189 336

CHEMICAL ABSTRACTS,
vol. 103, no. 3, 22 juillet 1985, page 38, résumé
no. 16618p, Columbus, Ohio, US; H. DREXLER et al.:
"Effects of calcium-blockade and converting-enzyme
inhibitor on regional blood flow in a conscious rat model
of heart failure", & KLIN.
WOCHENSCHR. 1985, 63(6), 262-7 000
PATENT ABSTRACTS OF JAPAN,
vol. 10, no. 103 (C-340)[2160], 18 avril 1986; &
JP-A-60 231 668 (TAKEDA YAKUHIN KOGYO
K.K.) 18-11-1985

Le dossier contient des informations techniques
présentées postérieurement au dépôt de la demande et
ne figurant pas dans le présent fascicule.

(73) Titulaire: SYNTHELABO, 58 rue de la Glacière,
F-75013 Paris(FR)

(72) Inventeur: Cavero, Icilio, 8, rue Gabriel Fauré,
F-94000 Creteil(FR)
Inventeur: Elkik, François, 12, rue Valentin Haüy,
F-75015 Paris(FR)
Inventeur: Hicks, Peter, 15, rue des Amandiers,
F-94230 Cachan(FR)
Inventeur: Muller, Jean-Claude, 4, Avenue de
l'Espérance, F-91390 Morsang s/Orge(FR)

(74) Mandataire: Thouret-Lemaitre, Elisabeth et al, Service
Brevets - SYNTHELABO 58, rue de la Glacière,
F-75621 Paris Cédex 13(FR)

## Description

La présente invention a pour objet des compositions pharmaceutiques contenant du diltiazem et un inhibiteur de l'enzyme de conversion de l'angiotensine.

Les inhibiteurs calciques prennent une place croissante dans le domaine de l'hypertension. Ils sont dorénavant recommandés comme traitement de première intention chez les hypertendus âgés dont on sait qu'ils sont très nombreux.

Dans cette indication, le diltiazem bénéficie d'une part de sa bonne tolérance, d'autre part de sa place de leader qu'il conquiert actuellement dans le domaine de l'angine de poitrine ; mais il pourrait être handicapé par sa relativement faible action vasodilatatrice périphérique.

L'hypertension est un domaine où la polythérapie est extrêmement fréquente.

Il apparaît cependant que les associations des inhibiteurs calciques avec des diurétiques et/ou des β-bloquants ne donnent pas entière satisfaction.

Les inhibiteurs de l'enzyme de conversion de l'angiotensine peuvent apporter une composante action vasodilatatrice périphérique tout à fait bénéfique.

H. Drexler et collégues, Klin. Wochenschr. (1985) 63: 262–267 ont étudié et composé les mérites respecifs du diltiazem et du Captopril dans des cas d'insuffisance cardiaque.

L'invention a donc pour objet les compositions pharmaceutiques contenant du diltiazem et un inhibiteur de l'enzyme de conversion de l'angiotensine.

Ce dernier peut être choisi parmi les composés suivants : captopril, enalapril, alacepril, enalaprilat, fentiapril, lisinopril, ramipril, perindopril, cilazapril, fosenopril, pivopril, quinilapril et zofenopril.

Les compositions de l'invention peuvent être utilisées pour le traitement de l'hypertension, l'insuffisance cardiaque et l'insuffisance coronaire.

La Demanderesse a étudié l'action des associations de diltiazem et d'inhibiteur de l'enzyme de conversion de l'angiotensine sur l'hypertension chez le rat spontanément hypertendu (SHR) vigile.

Le test utilisé est le suivant :

Un cathéter est installé à demeure dans l'artère caudale de SHR mâles, âgés de 12 mois, sous anesthésie légère à l'éther. Les animaux sont alors placés dans des cages individuelles leur permettant de se mouvoir librement et les cathéters connectés à des cellules de pression Statham P23Gc. Un dispositif spécial (Lavasseur J.E., Funk F.C., Patterson J.L. Jr., Physiological pressure transducer for microcirculatory studies. J Appl. Physiol. 27: 422-425, 1969) permet une perfusion intra-artérielle continue de soluté isotonique de chlorure de sodium (0,1 ml/kg/min) pendant toute la durée de l'expérimentation, cette perfusion évitant une éventuelle coagulation sans modifier la pression artérielle ou la fréquence cardiaque des animaux.

La pression artérielle est enregistrée en continu sur un polygraphe Grass (modèle 7D) à l'aide d'un préamplificateur Grass (modèle 7PI).

Après une période de stabilisation de 2 heures environ trois groupes d'animaux différents reçoivent par voie orale une dose de diltiazem (n=5/groupe), une dose d'inhibiteur de l'enzyme de conversion de l'angiotensine (n=5/groupe), ou l'association des deux doses de diltiazem et d'inhibiteur de l'enzyme de conversion de l'angiotensine (n=6/groupe). La pression systolique est mesurée avant l'administration des agents thérapeutiques puis 4 heures après l'administration d'un des composés ou de l'association de deux des agents thérapeutiques.

Les résultats sont donnés sous forme de moyennes de diminutions de la pression sanguine par rapport à la pression de base.

Dans le tableau suivant sont donnés les résultats obtenus, pour les composés administrés seuls et pour l'association de diltiazem et de captopril.

| COMPOSE | DOSE mg/kg p.o. | n | PAS initiale mmHg | Δ PAS (mmHg) | | | | |
|---|---|---|---|---|---|---|---|---|
| | | | | 0,5 | 1 h | 2 h | 3 h | 4 h |
| DILTIAZEM (DILT) | 25,0 | 5 | 209±4,3 | −15±3,2 | − 9±2,5 | − 2±5,8 | 0±5,2 | 1±5,8 |
| CAPTOPRIL (CAP) | 20,0 | 5 | 203±4,1 | −19±2,5 | −17±2,6 | −19±2,9 | −18±1,2 | −13±2,6 |
| DILT + CAP | 25/20 | 6 | 214±3,8 | −48±3,8* | −41±3,3* | −32±4,8* | −24±5,4 | −23±5,9 |

*: P < 0,05 par rapport au DILT ou CAP

Les résultats montrent que l'association de diltiazem et de captopril produit un effet antihypertenseur significativement plus important que la somme des effets engendrés par chacun des composés administrés isolément.

Il existe donc une synergie entre les effets antihypertenseurs du diltiazem et de l'inhibiteur de l'enzyme de conversion de l'angiotensine cité, par unité de prise.

Les compositions pharmaceutiques de l'invention peuvent contenir de 10 à 360 mg de diltiazem et de 1 à 100 mg d'un inhibiteur de l'enzyme de conversion de l'angiotensine cité, par unité de prise.

Les compositions pharmaceutiques de l'invention peuvent être présentées sous toute forme appropriée pour l'administration par voie orale ou parentérale, en association avec tout excipient approprié.

Les compositions pharmaceutiques de l'invention peuvent être utilisées pour le traitement de l'hypertension.

La posologie quotidienne est telle que l'on administre de 10 à 240 mg de diltiazem et de 1 à 100 mg de l'un des inhibiteurs de l'enzyme de conversion de l'angiotensine.

**Revendications**

1. Compositions pharmaceutiques contenant une association de diltiazem et d'un inhibiteur de l'enzyme de conversion de l'angiotensine.

2. Compositions pharmaceutiques contenant du diltiazem et un inhibiteur de l'enzyme de conversion de l'angiotensine choisi parmi les composés suivants: captopril, enalapril, alacepril, enalaprilate, fentiapril, lisinopril, ramipril, perindopril, cilazapril, fosinopril, pivopril, quinilapril et zofenopril.

3. Compositions pharmaceutiques contenant du diltiazem et du captopril.

4. Composition pharmaceutique contenant de 10 à 360 mg de diltiazem et de 5 à 50 mg de captopril.

5. Compositions pharmaceutiques contenant du diltiazem et de l'enalapril.

6. Compositions pharmaceutiques contenant du diltiazem et du fosinopril.

**Patentansprüche für die Vertragsstaaten AT, BE, CH, DE, FR, GB, IT, LI, LU, NL, SE**

1. Pharmazeutische Zusammensetzungen, die eine Kombination von Diltiazem und einem Inhibitor des Umwandlungsenzyms des Angiotensins enthalten.

2. Pharmazeutische Zusammensetzungen, die Diltiazem und einen Inhibitor des Umwandlungsenzyms des Angiotensins, ausgewählt aus folgenden Verbindungen, enthalten: Captopril, Enalapril, Alacepril, Enalaprilat, Fentiapril, Lisinopril, Ramipril, Perindopril, Cilazapril, Fosenopril, Pivopril, Quinilapril und Zofenopril.

3. Pharmazeutische Zusammensetzungen, die Diltiazem und Captopril enthalten.

4. Pharmazeutische Zusammensetzung, die 10 bis 360 mg Diltiazem und 5 bis 50 mg Captopril enthält.

5. Pharmazeutische Zusammensetzungen, die Diltiazem und Enalapril enthalten.

6. Pharmazeutische Zusammensetzungen, die Diltiazem und Fosinopril enthalten.

**Claims for the contracting states: AT, BE, CH, DE, FR, GB, IT, LI, LU, NE, SE**

1. Pharmaceutical compositions containing a combination of diltiazem and an angiotensin-converting enzyme inhibitor.

2. Pharmaceutical compositions containing diltiazem and an angiotensin-converting enzyme inhibitor chosen from the following compounds: captopril, enalapril, alacepril, enalaprilat, fentiapril, lisinopril, ramipril, perindopril, cilazapril, fosenopril, pivopril, quinilapril and zofenopril.

3. Pharmaceutical compositions containing diltiazem and captopril.

4. Pharmaceutical compositions containing from 10 to 360 mg of diltiazem and from 5 to 50 mg of captopril.

5. Pharmaceutical compositions containing diltiazem and enalapril.

6. Pharmaceutical compositions containing diltiazem and fosinopril.

3